# FASCICULE DE BREVET EUROPEEN

(11) **EP 2 227 186 B1**
(45) Date de publication et mention de la délivrance du brevet: **11.09.2019**
(21) Numéro de dépôt: 08852999.5
(22) Date de dépôt: 21.11.2008
(51) Int. Cl.: A61M 25/10, A61F 2/06, A61F 2/91, A61F 2/95, A61F 2/958, A61M 25/06

(54) **DISPOSITIF D'IMPLANTATION D'UNE PROTHÈSE VASCULAIRE**
VORRICHTUNG ZUR IMPLANTATION EINER GEFÄSSPROTHESE
DEVICE FOR IMPLANTING A VASCULAR PROSTHESIS

(30) Priorité: 23.11.2007 FR 0759269
(43) Date de publication de la demande: 15.09.2010
(73) Titulaire: Assistance Publique Hôpitaux de Marseille, 13354 Marseille (FR); Université d'Aix-Marseille, 13284 Marseille Cedex 07 (FR)
(72) Inventeur: ALIMI, Yves, F-06000 Nice (FR); GARITEY, Vincent, F-06000 Nice (FR); RAMOS CLAMOTE, Joachim, F-06000 Nice (FR)
(74) Mandataire: Hautier, Nicolas
(86) Numéro de dépôt international: PCT/EP2008/065957
(87) Numéro de publication internationale: WO 2009/065917

(56) Documents cités:
- WO-A-00/66007
- WO-A-99/13808
- WO-A2-03/075976
- US-A- 4 983 166
- US-A- 5 972 017
- US-A1- 2007 016 241
- US-B1- 7 141 060

## Description

L'invention porte sur un dispositif d'implantation d'une prothèse vasculaire. Plus particulièrement elle concerne un cathéter de dilatation associé à un ballon, le ballon étant destiné à permettre à un stent d'impacter une prothèse vasculaire dans un vaisseau. L'invention trouvera notamment pour application la réalisation d'une implantation de prothèse vasculaire au droit d'une ponction effectuée dans un vaisseau et à travers laquelle est introduite la prothèse. De manière particulièrement avantageuse, l'invention s'appliquera à la réalisation de pontage ou encore à la fermeture d'une voie d'accès vasculaire.

De manière connue, notamment du document US 5 972 017, l'implantation d'une prothèse vasculaire au droit d'une ponction transpariétale à travers laquelle est introduite la prothèse, est obtenue en positionnant la prothèse au droit de la ponction puis en déployant un stent contenu dans la prothèse. Un ballon alimenté en fluide par un cathéter de dilatation permet d'impacter l'ensemble stent - prothèse vasculaire dans la paroi du vaisseau.

Une telle implantation de prothèse engendre de nombreuses difficultés. Notamment la position de la prothèse par rapport au vaisseau doit être parfaitement contrôlée. De plus, la stabilité et l'étanchéité de la liaison entre la prothèse et le vaisseau doivent également être maîtrisées avec une très bonne précision.

Par ailleurs, lorsque l'opération est réalisée sans clampage du vaisseau, cette mise en position de la prothèse s'avère plus difficile notamment du fait de l'écoulement du sang. En outre, en l'absence de clampage l'introduction de la prothèse et sa mise en position et l'étanchéité de la liaison doivent être effectuées de manière précise, rapide et simple sous peine de perte sanguine majeure voire mortelle.

Il serait ainsi particulièrement appréciable de disposer d'un dispositif d'implantation de prothèse vasculaire permettant d'améliorer la précision et/ou la simplicité et/ou la rapidité des procédés connus d'implantation de prothèse vasculaire.

A cet effet, la présente invention comme décrite dans la revendication 1, prévoit un système d'implantation d'une prothèse au sein d'un vaisseau, comportant un cathéter d'introduction et un dispositif d'implantation comprenant un ballon et un cathéter de dilatation pour alimenter le ballon en fluide, le cathéter de dilatation comprenant
une extrémité proximale, une extrémité distale et une partie distale destinée à être introduite dans le vaisseau, caractérisé en ce que la partie distale comprend un coude ainsi qu'une portion amont et une portion aval contiguës au coude et disposées entre le coude et respectivement l'extrémité proximale et l'extrémité distale et en ce que le ballon est porté entièrement par la portion aval, le dispositif d'implantation étant destiné à coulisser depuis l'extrémité proximale jusqu'à l'extrémité distale du cathéter d'introduction, le dispositif d'implantation comportant une prothèse et/ou un stent à l'intérieur duquel est logé le ballon.

Ainsi un dispositif d'implantation selon l'invention permet de recevoir sur sa portion aval un ballon pouvant s'étendre sur une direction sensiblement parallèle à la dimension longitudinale du vaisseau. Le ballon peut ainsi épouser la forme de la paroi interne du vaisseau sur une distance longitudinale suffisamment importante pour couvrir la totalité des dimensions longitudinales de la prothèse et du stent disposé entre la prothèse et le ballon.

La forme particulière du cathéter de dilatation offre une grande liberté de choix concernant les dimensions et le matériau du ballon. Elle permet ainsi de s'affranchir des contraintes habituelles associées aux implantations réalisées au droit d'une ponction.

En outre, le cathéter de dilatation s'étend le long du ballon et peut traverser ce dernier de part en part. Il permet ainsi d'assurer un bon maintien du ballon sur le cathéter de dilatation. Ce maintien offre un contrôle de la position et du maintien du ballon par déplacement du cathéter de dilatation tout au long des étapes de positionnement du ballon intervenant avant et après la dilatation de ce dernier.

Le dispositif d'implantation selon l'invention permet ainsi d'améliorer la précision du positionnement du ballon du stent et de la prothèse vasculaire au cours du procédé d'implantation de la prothèse au droit de la ponction.

Or, il s'est avéré que le positionnement et le maintien en position dans le vaisseau de la prothèse et du stent conditionnent directement l'étanchéité et la robustesse de la liaison entre la prothèse et le vaisseau.

Ainsi un dispositif d'implantation selon l'invention contribue à augmenter significativement la qualité des implantations de prothèses vasculaires.

Par ailleurs, l'utilisation d'un dispositif d'implantation selon l'invention s'avère simple à manipuler. Un tel dispositif permet de limiter la durée et la difficulté des procédés connus d'implantation de prothèse vasculaire.

Un dispositif d'implantation ne comprenant pas l'invention permet également de recevoir un ballon en partie sur sa portion aval et en partie sur sa portion amont. Cette variante de réalisation permet de simplifier considérablement les opérations à effectuer pour positionner précisément le ballon. Elle contribue ainsi à réduire significativement la durée et la complexité d'une opération d'implantation d'une prothèse.

De manière facultative, un dispositif d'implantation selon l'invention pourra présenter l'une quelconque des caractéristiques suivantes :
- les portions amont et aval forment un angle compris entre 0° et 30°.
- le coude présente sensiblement une forme de U.
- les portions amont et aval sont sensiblement parallèles.
- le cathéter de dilatation est élastique en particulier au niveau du coude de manière à ce que les portions amont et aval puissent être mutuellement approchées ou écartées.
- le cathéter de dilatation comporte une première lumière s'étendant entre son extrémité proximale et le coude.
- la première lumière est sensiblement rectiligne.
- cette première lumière est apte à recevoir un premier guide.
- le cathéter de dilatation comporte une deuxième lumière s'étendant entre l'extrémité proximale et l'extrémité distale du cathéter de dilatation, la deuxième lumière étant conformée pour recevoir un second guide.
- le coude et/ou l'extrémité distale du cathéter de dilatation présente un embout profilé en forme de cône ou de sphère prolongeant la portion aval.
- il comporte une prothèse et/ou un stent à l'intérieur duquel est logé le ballon.

En outre, on prévoit selon l'invention un système d'implantation d'une prothèse dans un vaisseau comportant un dispositif d'implantation selon le dispositif précédent comprenant le ballon étant entièrement porté par la portion aval du cathéter de dilatation et un cathéter d'introduction, comprenant une extrémité proximale et une extrémité distale, le dispositif d'implantation étant destiné à coulisser depuis l'extrémité proximale jusqu'à l'extrémité distale du cathéter d'introduction.

Le système d'implantation selon l'invention pourra facultativement présenter l'une quelconque des caractéristiques suivantes :
- le cathéter d'introduction comporte des moyens d'étanchéité et de stabilisation agencés de manière à permettre une liaison stable et étanche entre le cathéter d'introduction et le vaisseau.
- les moyens d'étanchéité et de stabilisation comportent un bossage et une gorge disposée entre le bossage et l'extrémité distale du cathéter d'introduction,
- les moyens d'étanchéité et de stabilisation sont élastiques et agencés pour épouser les parois d'un orifice approximativement circulaire.
- la gorge est constituée par un matériau élastique apte à épouser le profil du passage pratiqué à travers la paroi du vaisseau.
- les moyens d'étanchéité et de stabilisation sont indéformables et inamovibles.
- l'extrémité distale du cathéter d'introduction comporte un chanfrein ou une paroi externe s'évasant en direction de l'extrémité proximale du cathéter d'introduction.
- il comprend un cathéter de largage et/ou un dispositif de pousse agencés de manière à faire coulisser le dispositif d'implantation depuis l'extrémité proximale du cathéter d'introduction jusqu'à l'extrémité distale de ce dernier.

La présente invention et ses avantages seront mieux compris à la lumière de la description détaillée qui va suivre et au regard des dessins annexés sur lesquels :
- la figure 1 représente les différents cathéters et un dispositif de pousse permettant d'introduire un dispositif d'implantation selon l'invention à l'intérieur d'un vaisseau,
- les figures 2 et 3 représentent des vues respectivement en perspective et en coupe longitudinale de l'extrémité distale d'un exemple particulier de cathéter d'introduction,
- les figures 4 à 19 représentent les différentes étapes de l'implantation d'une prothèse selon un exemple de réalisation de l'invention,
- la figure 20 représente un dispositif d'implantation selon un exemple de réalisation de l'invention,
- les figures 21 et 22 représentent un exemple particulier de cathéter d'introduction dans deux configurations différentes,
- les figures 23 et 24 représentent respectivement une vue longitudinale et une vue en coupe transversale d'un exemple particulier de réalisation de l'invention,
- la figure 25 représente une vue en coupe longitudinale d'un autre exemple particulier de réalisation de l'invention,
- la figure 26 représente trois vues en coupe transversale d'un exemple de cathéter de dilatation selon l'invention, chacune des vues étant prise en une section différente du cathéter.

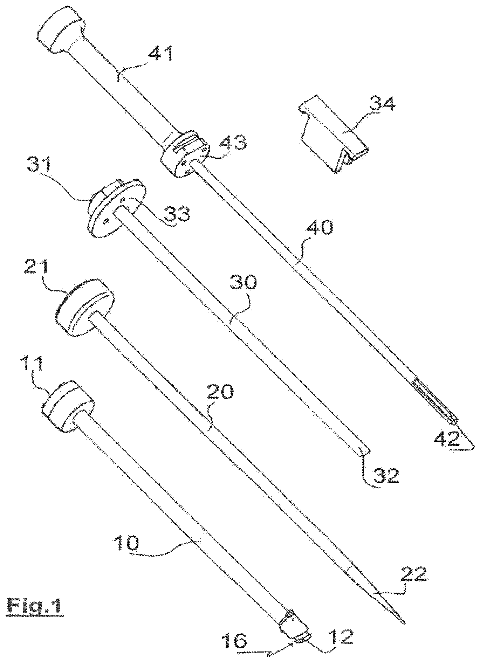

Un premier exemple d'un système d'implantation selon l'invention va maintenant être décrit en référence à la figure 1. Dans cet exemple, le système d'implantation comprend un cathéter d'introduction 10, des moyens de pénétration 20, un cathéter de largage 30, un dispositif de pousse 40 et un dispositif d'implantation 120 contenu dans le cathéter de largage.

Le cathéter d'introduction 10 comprend un corps tubulaire délimité par une extrémité proximale 11 et une extrémité distale 12. L'extrémité distale 12 est destinée à être introduite dans le vaisseau 1 au travers d'un passage transpariétal pratiqué par ponction du vaisseau 1.

De manière avantageuse, l'extrémité proximale 11 est équipée d'une valve hémostatique, et d'une voie d'entrée avec robinet luer destinée à permettre l'injection de produit tel que du sérum hépariné.

L'extrémité distale 12 est taillée en biseau pour une meilleure pénétration jusque dans la zone d'implantation. A proximité de son extrémité distale 12, le cathéter d'introduction 10 comporte des moyens d'étanchéité et de stabilisation 16 du cathéter d'introduction 10 sur le vaisseau 1. Ces moyens d'étanchéité et de stabilisation 16 sont destinés à être retirés du vaisseau 1 une fois la prothèse 110 implantée. Ils assurent la mise en position, le maintien en position et l'étanchéité du cathéter d'introduction 10 par rapport au vaisseau 1.

Selon un mode de réalisation privilégié, ils comprennent un bossage 13 disposé à quelques millimètres en amont de l'extrémité distale 12 du cathéter d'introduction 10. Le bossage 13 est agencé de manière à faire office de butée entre le vaisseau 1 et le cathéter d'introduction 10 lors de l'introduction de ce dernier dans le vaisseau 1. Ainsi, le déplacement du cathéter d'introduction 10 au travers du vaisseau 1 est stoppé lorsque le bossage 13 entre au contact du pourtour du passage transpariétal. La position du bossage 13 par rapport à l'extrémité distale 12 du cathéter d'introduction 10 détermine la longueur du cathéter d'introduction 10 qui peut pénétrer au sein du vaisseau 1. La mise en butée du bossage 13 sur le vaisseau 1 permet ainsi de positionner précisément le cathéter d'introduction 10.

Avantageusement, l'extrémité distale du bossage 13 est comprise dans un plan parallèle au plan défini par la section de l'extrémité distale 12 du cathéter d'introduction 10 taillée en biseau. Cette configuration du bossage 13 permet d'améliorer la pénétration, la maniabilité ainsi que le maintien du cathéter d'introduction 10 sur le vaisseau 1.

Préférentiellement, les moyens d'étanchéité et de stabilisation 16 présentent une gorge 14 contiguë au bossage 13 et située entre ce dernier et l'extrémité distale 12 du cathéter d'introduction 10. La gorge 14 est également contenue dans un plan parallèle au plan défini par la section de l'extrémité distale 12 du cathéter d'introduction 10 taillée en biseau. La gorge 14 est agencée de manière à coopérer avec le pourtour du passage transpariétal pratiqué dans le vaisseau 1. Le vaisseau 1 présentant une certaine élasticité la pénétration du cathéter d'introduction 10 dans le passage transpariétal génère des contraintes sensiblement radiales sur le pourtour de ce dernier. Ces contraintes diminuent lorsque le pourtour se loge dans la gorge 14 puisque la section du cathéter d'introduction 10 au niveau de la gorge 14 est plus petite qu'entre la gorge 14 et l'extrémité distale 12 du cathéter d'introduction 10.

Une fois logé dans la gorge 14, le pourtour occupe alors une position d'équilibre stable qui contribue au maintien de la liaison entre le cathéter d'introduction 10 et le vaisseau 1.

Ainsi la combinaison du bossage 13 et de la gorge 14 assure une mise en position et un maintien en position particulièrement stable du cathéter d'introduction 10 sur le vaisseau 1. En outre cette combinaison assure une étanchéité efficace entre le pourtour du passage transpariétal et le cathéter d'introduction 10.

Par ailleurs, cette mise en position, ce maintien en position et cette étanchéité sont effectués de manière particulièrement aisée et rapide par l'opérateur. En effet l'insertion du pourtour dans la gorge 14 est obtenue de manière quasiment automatique et instantanée lorsque le bossage 13 parvient en butée sur la paroi externe du vaisseau 1.

Ces moyens d'étanchéité et de stabilisation 16 ne nécessitent ni actionnement d'organe amovible ni alimentation de circuit hydraulique. En outre ils sont formés d'un seul tenant avec le cathéter d'introduction 10 et s'avèrent particulièrement robustes. Or, il est particulièrement important de garantir une étanchéité fiable entre le cathéter d'introduction 10 et le vaisseau 1. Plus les moyens assurant l'étanchéité sont robustes et simples à manipuler, meilleure est la fiabilité de l'étanchéité lors de l'implantation de la prothèse 110.

Le cathéter d'introduction 10 est particulièrement facile et économique à fabriquer. Le bossage 13 et la gorge 14 peuvent par exemple être venus de matière, ou réalisés par moulage ou surmoulage, ou encore rapportés sur le cathéter d'introduction 10.

Nous désignerons à partir de maintenant par implant l'ensemble formé par la prothèse vasculaire et le stent.

Les moyens de stabilisation et d'étanchéité du cathéter d'introduction 10 contribuent, tout comme le dispositif d'implantation décrit par la suite, à améliorer significativement la rapidité et la simplicité des procédés connus d'implantation de l'implant. Cependant, l'implémentation de ces moyens de stabilisation et d'étanchéité d'une part et du dispositif d'implantation d'autre part sont parfaitement indépendantes.

On peut notamment prévoir de modifier la structure des moyens d'étanchéité et de stabilisation 16.

Ainsi, selon des variantes de réalisation, ces moyens ne comprennent ni bossage 13 ni gorge 14 inamovible et indéformable, mais un organe en matériau à mémoire de forme, un élastomère ou un ballonnet gonflable ou encore une combinaison de tels organes et d'un tel ballonnet.

L'organe ou le ballonnet permet de créer de manière contrôlée un bossage 13 et/ou une gorge 14 de manière à ce que lorsque le cathéter d'introduction 10 pénètre dans le vaisseau 1, l'organe et/ou le ballonnet se déforme et enserre la paroi du pourtour du passage transpariétal. Ils assurent ainsi stabilité et étanchéité entre le cathéter d'introduction 10 et l'artère.

Selon un exemple particulier de réalisation du cathéter d'introduction 10, ce dernier comporte à son extrémité distale 12 des moyens de pénétration. Ces moyens de pénétration vont permettre de dilater l'orifice de ponction, ils se présentent sous la forme d'un nez conique 20.

Ils s'étendent sensiblement selon la direction longitudinale du corps tubulaire et, de manière avantageuse sont flexibles. Ces moyens de pénétration présentent une extrémité distale 22 et une extrémité proximale 21. Ils présentent une portion cylindrique tubulaire s'étendant depuis l'extrémité proximale 21 en direction de l'extrémité distale 22 et une portion conique s'étendant depuis l'extrémité distale jusqu'à la portion cylindrique. Ainsi, l'extrémité distale 22 forme sensiblement la pointe d'un cône et la portion conique s'évase jusqu'à la portion cylindrique. Ils sont conformés de manière à ce que la pointe du cône soit introduite dans l'extrémité proximale 11 du cathéter d'introduction 10 et à coulisser le long du corps tubulaire jusqu'à dépasser de l'extrémité distale 12 du cathéter d'introduction 10. Le diamètre extérieur de l'extrémité distale est conformé pour que les moyens de pénétration puissent être aisément introduits dans la lumière du vaisseau 1. La section de l'extrémité proximale 21 des moyens de pénétration est conformée de manière à stopper le coulissement des moyens de pénétration à l'intérieur du cathéter d'introduction 10 lorsque ces derniers dépassent suffisamment de l'extrémité distale 12 du cathéter d'introduction 10.

Les moyens de pénétration sont agencés de manière à élargir la section du passage transpariétal au fur et à mesure de leur introduction dans le vaisseau 1. Ils permettent ainsi de faciliter l'introduction et le positionnement de l'extrémité distale 12 du cathéter d'introduction 10 dans le vaisseau 1.

En outre, les moyens de pénétration présentent une lumière les traversant de part en part depuis l'extrémité proximale 21 jusqu'à leur extrémité distale 22. Cette lumière est destinée à recevoir au moins un guide le long duquel les moyens de pénétration peuvent être guidés par coulissement.

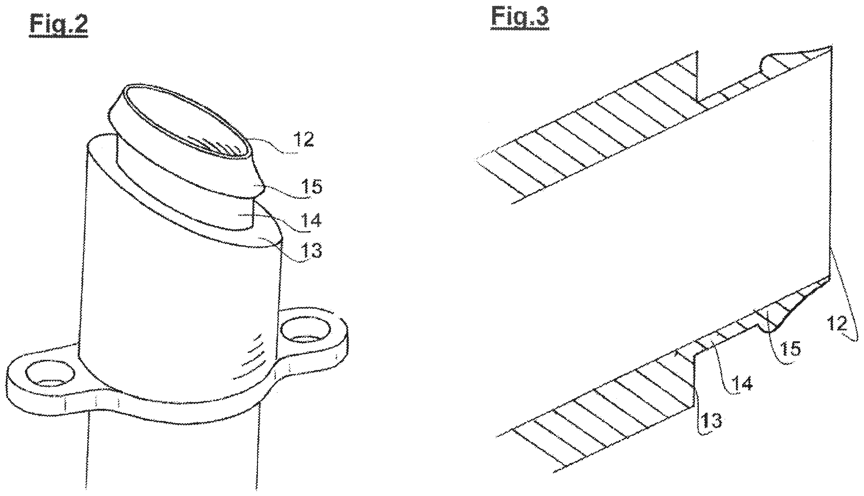

Avantageusement, le cathéter d'introduction 10 présente un profilé 15 dont la forme s'évase depuis l'extrémité distale 12 en direction de l'extrémité proximale 11. Ce profilé 15 présente par exemple une forme sensiblement tronconique et/ou chanfreinée. Il peut également présenter une courbure dont la pente varie comme illustré sur le mode de réalisation des figures 2 et 3. Il est agencé pour faciliter l'insertion du cathéter d'introduction 10 dans le vaisseau 1 à travers le passage transpariétal.

Avantageusement, le profilé 15 s'étend depuis l'extrémité distale 12 jusqu'à la gorge 14. Il présente en outre un tronçon arrondi à sa jonction avec la gorge 14 afin d'assurer un guidage continu et progressif du pourtour du passage transpariétal depuis l'extrémité distale 12 jusqu'à la gorge 14.

Il se situe dans le prolongement du corps du cathéter d'introduction 10. Il présente une pente supérieure à celle du nez conique 20. De manière préférée, cette pente est supérieure de 10 à 70 degrés à celle d'un nez conique 20.

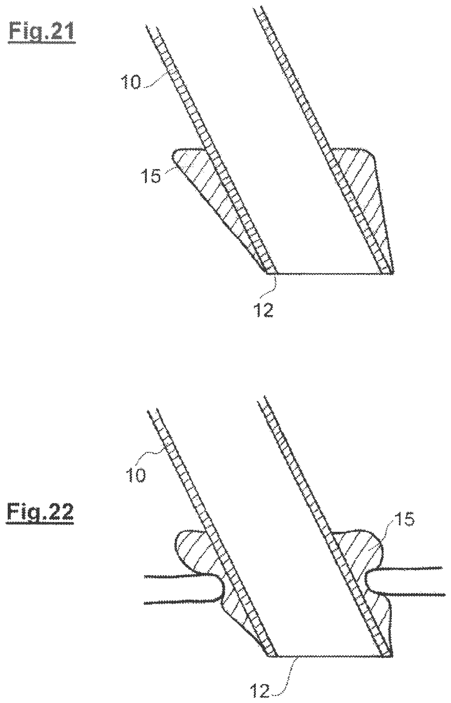

Dans le mode particulier de réalisation représenté en détail sur les figures 21 et 22, le profilé 15 est constitué en tout ou partie d'un matériau souple et élastique tel que du silicone ou tout autre élastomère biocompatible. Sur la figure 21 le profilé 15 est dans sa position de repos. Sur la figure 22, l'extrémité distale 12 du cathéter d'introduction 10 est insérée dans la paroi du vaisseau 1. Le profilé 15 se déforme alors et épouse la forme du pourtour de l'ouverture pratiquée dans le vaisseau.

L'agencement et le matériau du profilé 15 contribuent à améliorer la mise en position du cathéter d'introduction 10 sur le vaisseau 1. Ils favorisent également l'adaptation de l'extrémité distale 12 du cathéter d'introduction 10 au pourtour du passage transpariétal.

Ce profilé 15 améliore par conséquent significativement l'étanchéité de la liaison entre le cathéter d'introduction 10 et le vaisseau 1.

Le profilé 15 de l'extrémité distale 12 du cathéter d'introduction 10 contribue également à faciliter et à simplifier l'implantation de la prothèse 110. Ce profilé 15 est techniquement indépendant des moyens de stabilisation et d'étanchéité précédemment décrits et du cathéter de dilatation 120 qui sera détaillé par la suite.

Le système d'implantation comporte également un cathéter de largage 30 apte à être inséré dans le cathéter d'introduction 10. Le cathéter de largage 30 présente une extrémité proximale 31, une extrémité distale 32 et un corps tubulaire s'étendant entre l'extrémité proximale 31 et l'extrémité distale 32.

Les sections extérieures de l'extrémité distale 32 et du corps tubulaire du cathéter de largage 30 sont agencées de manière à ce que ce dernier puisse coulisser à l'intérieur du corps du cathéter d'introduction 10 en étant introduit par l'extrémité proximale 11 de ce dernier.

Avantageusement, le jeu radial entre le cathéter de largage 30 et le cathéter d'introduction 10 est suffisamment faible pour permettre un guidage précis du cathéter de largage 30 lors de son coulissement dans le corps du cathéter d'introduction 10.

Le cathéter de largage 30 comporte à proximité de son extrémité proximale une butée 33 destinée à coopérer avec l'extrémité proximale 11 du cathéter d'introduction 10 pour stopper le coulissement du cathéter de largage 30 en direction de l'extrémité distale 12 du cathéter d'introduction 10. Le système d'implantation est agencé de sorte que lorsque ladite butée 33 entre en contact de l'extrémité proximale 11 du cathéter d'introduction 10, les extrémités distales des cathéters d'introduction et de largage sont disposées sensiblement en regard.

Le cathéter de largage 30 présente une lumière s'étendant depuis son extrémité proximale 31 jusqu'à son extrémité distale 32. Cette lumière est agencée de manière à loger un dispositif d'implantation. L'extrémité distale du cathéter de largage présente un biseau identique à celui du cathéter d'introduction sur lequel il est possible de l'aligner grâce à des repères ou des butées angulaires. Par ailleurs le système comprend un dispositif de pousse 40 ayant pour fonction d'éjecter le dispositif d'implantation hors du dispositif de largage en le poussant, au moins partiellement au delà de l'extrémité distale 32 du cathéter de largage 30 et du cathéter d'introduction 10. Ainsi poussé, le dispositif d'implantation peut être positionné, au moins en partie dans le vaisseau 1 à implanter. A cet effet le dispositif de pousse 40 comporte une extrémité proximale 41 associée à des moyens de préhension destinés à être manipulés par un opérateur. Il comporte par ailleurs une extrémité distale 42 destinée à être introduite dans le corps du cathéter de largage 30 depuis l'extrémité proximale 31 de ce dernier jusqu'à entrer en contact du dispositif d'implantation. La dimension longitudinale du dispositif de pousse 40 est sensiblement supérieure à la dimension longitudinale du cathéter de largage 30 et à celle du cathéter d'introduction 10.

Avantageusement, le dispositif de pousse 40 comprend des moyens de réglage permettant de contrôler la distance sur laquelle le dispositif d'implantation est poussé hors du cathéter de largage 30. Ces moyens de réglage comprennent une butée 43 destinée à entrer au contact de l'extrémité proximale 31 du cathéter de largage 30. Ils comprennent également une entretoise, ou cale 34, disposée de manière amovible entre ladite butée 43 et l'extrémité proximale 31 du cathéter de largage 30.

Le système d'implantation comporte une prothèse vasculaire 110 destinée à être déployée une fois positionnée dans le vaisseau 1.

La prothèse vasculaire 110 peut être droite ou bifurquée. Elle comporte un tronçon principal ou corps 111 destiné à être positionné à l'intérieur du vaisseau 1 implanté. Elle comporte également dans le cas d'un pontage une ou plusieurs jambes 112 dont l'extrémité amont débouche dans la portion principale de la prothèse 110 et dont l'extrémité aval est destinée à être connectée fluidiquement avec une portion de vaisseau 1 située en aval de la zone implantée.

Dans le cas du pontage d'un vaisseau 1 sténosé, la prothèse 110 est implantée de manière à ce que la sténose 2 soit située entre les extrémités amont et aval de chacune des jambes 112. Ainsi ce dispositif permet au sang de s'écouler par les jambes 112 formant le pontage et également au travers de la sténose 2.

La connexion entre l'extrémité aval des jambes 112 et un vaisseau peut par exemple être réalisée par suture.

Le dispositif d'implantation comporte également un stent 130 logé à l'intérieur de la prothèse vasculaire 110. De manière connue, le stent 130 peut notamment assurer la fonction de maintien de la prothèse 110 au niveau de la ponction du vaisseau 1. Le stent 130 a également pour rôle de contribuer à la création de l'étanchéité entre la prothèse vasculaire 110 et le vaisseau 1.

Le stent de l'implant peut être à mémoire de forme ou non, et il peut être situé dans le diamètre interne de la prothèse vasculaire ou noyé en son sein.

Avantageusement le stent 130 est auto extensible. Cette faculté peut notamment provenir de la nature de son matériau. A cet effet on pourra prévoir de constituer le stent 130 en un alliage à mémoire de forme.

Le stent 130 est lié à la prothèse vasculaire 110. Après déploiement du stent 130, la partie de la prothèse 110 introduite dans le vaisseau 1 se retrouve prise en sandwich et immobilisée entre la paroi externe du stent 130 et la paroi interne du vaisseau 1, ce qui a pour effet de créer l'étanchéité entre l'implant et le vaisseau.

Le dispositif d'implantation comprend également un ballon 128 ainsi qu'un cathéter de dilatation 120.

Avant montage dans le cathéter de largage 30, le cathéter de dilatation 120 et le ballon 128 sont positionnés dans le stent 130. Le stent 130 est ensuite comprimé et, selon un mode de réalisation particulier, maintenu par des liens. Ces liens peuvent être défaits depuis l'extrémité proximale 31 du cathéter de largage 30 afin de permettre au stent 130 de se déployer.

Le ballon 128 a pour fonction de se dilater afin d'impacter l'implant dans le vaisseau 1. Le stent 130 peut ainsi se déployer pleinement et maintenir fermement la prothèse vasculaire 110 au contact du vaisseau 1 assurant par la même occasion une étanchéité efficace.

Le cathéter de dilatation 120 présente une extrémité proximale et une extrémité distale 124 et un corps s'étendant entre l'extrémité proximale et l'extrémité distale 124.

Le cathéter de dilatation 120 présente une partie distale destinée à pénétrer au sein du vaisseau 1. Cette partie distale comprend un coude 123, une portion amont 121 et une portion aval 122. La portion amont 121 est contiguë au coude 123 et est disposée entre ce dernier et l'extrémité proximale du cathéter de dilatation 120. La portion aval 122 est contiguë au coude 123 et est disposée entre ce dernier et l'extrémité distale 124 du cathéter de dilatation 120. Les portions amont 121 et aval 122 sont sensiblement rectilignes.

Le ballon 128 est porté par la portion aval 122. Le cathéter de dilatation 120 présente un canal permettant d'alimenter le ballon 128 en fluide. Avantageusement il présente une forme sensiblement longitudinale et s'étend le long de la portion aval 122. Il est traversé de part en part par le cathéter de dilatation 120.

De manière préférée le ballon 128 est lié au cathéter de dilatation 120 par les deux points de liaisons correspondant aux zones dans lesquelles le cathéter de dilatation 120 traverse le ballon 128. Le ballon 128 est ainsi particulièrement bien maintenu sur le cathéter de dilatation 120.

Le coude 123 est agencé de manière à ce que la portion aval 122 forme un retour en direction de la portion amont 121. Le coude 123 présente sensiblement une forme de U. Les portions amont et aval forment entre elles un angle compris entre 0° et 45° et plus particulièrement compris entre 0° et 20°. Avantageusement cet angle est proche de 0° de manière à ce qu'en position de repos les portions amont et aval s'étendent selon des directions sensiblement parallèles.

Le cathéter de dilatation 120 est agencé pour que, lorsqu'il est disposé dans le cathéter d'introduction 10, la portion aval 122 soit sensiblement repliée le long de la portion amont 121. Les portions amont 121 et aval 122 s'étendent sensiblement selon la direction longitudinale du cathéter d'introduction 10. Elles sont disposées côte à côte. Le coude 123 est alors disposé, par rapport au cathéter d'introduction 10, en aval des portions amont 121 et aval 122. Lorsque le dispositif est à l'intérieur du cathéter d'introduction 10, le ballon 130, porté par la portion aval 122, est disposé le long de la portion amont 121.

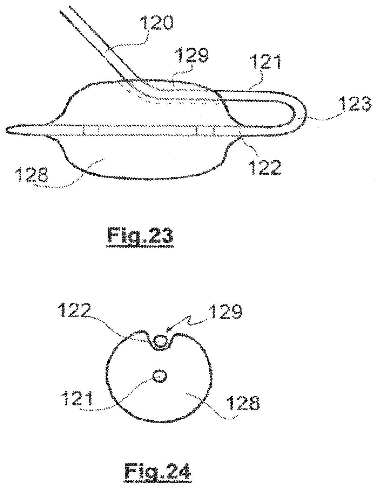

De manière facultative le ballon 128 définit, en configuration dilatée, une rainure 129. Cette rainure 129 est illustrée sur les dispositifs d'implantation représentés sur les figures 23 et 24. Le ballon 128 est agencé de manière à ce que la rainure 129 soit disposée au regard de la portion amont 121 et s'étende sensiblement parallèlement à cette dernière. En outre la rainure 129 est conformée de sorte à accueillir en son sein la portion amont 121 lorsque le ballon se dilate. Ainsi, la rainure 129 s'étend sur une distance suffisante pour loger l'ensemble de la portion amont au contact du ballon. Elle s'étend sur sensiblement la moitié de la longueur longitudinale du ballon. La profondeur de la rainure 129 est déterminée de sorte que la portion amont 121 ne forme pas saillie sur l'enveloppe externe du ballon. Par ailleurs, la rainure 129 présente une largeur suffisante pour que la portion amont 121 s'insère automatiquement dans la rainure 129 lors de la dilatation du ballon128.

Avantageusement, une telle rainure 129 permet au dispositif d'implantation de ne pas présenter de saillie qui pourrait déformer la paroi du vaisseau lorsque le ballon est dilaté. Le dispositif d'implantation épouse ainsi parfaitement la paroi interne du vaisseau.

Le cathéter de dilatation 120 est flexible, notamment au niveau du coude 123.

Ainsi lorsque la partie distale est introduite dans le vaisseau 1 à implanter par le passage transpariétal, la portion amont et la portion aval qui porte à elle seule le ballon s'orientent dans des directions sensiblement parallèles à la direction principale 3 du vaisseau 1. On désigne par la suite direction principale 3, la direction définie par le flux sanguin s'écoulant dans le vaisseau 1.

La flexibilité du cathéter de dilatation 120 et l'inclinaison en biseau de l'extrémité distale 12 du cathéter d'introduction 10 contribuent à disposer la portion amont 121 selon la direction principale 3 du vaisseau 1.

Le cathéter de dilatation 120 est agencé de manière à ce que son extrémité proximale puisse dépasser des cathéters d'introduction et de largage.

La portion amont et la portion aval du cathéter de dilatation 120 sont conformées de sorte que, grâce à une simple manoeuvre de recul dans le cathéter d'introduction d'une distance prédéterminée, le ballon 128 se positionne sensiblement au droit du passage transpariétal au travers duquel le cathéter d'introduction 10 est inséré dans le vaisseau 1. Plus précisément, le centre du ballon 128 est disposé au droit du passage transpariétal.

Ainsi le ballon 128 peut épouser la forme de la paroi interne du vaisseau 1 selon une distance longitudinale suffisamment importante pour recouvrir la totalité de la dimension longitudinale des parois internes de la prothèse vasculaire 110 et du stent 130. La dimension longitudinale du ballon 128 peut ainsi être adaptée pour impacter le stent 130 sur l'ensemble de la dimension longitudinale de ce dernier.

Avantageusement, le ballon 128 s'étend sensiblement sur la totalité de la longueur de la portion aval 122. La portion aval porte à elle seule le ballon.

Le cathéter de dilatation 120 comporte également une première lumière 141 apte à accueillir un premier guide 5. Le premier guide 5 présente une extrémité proximale et une extrémité distale. Cette première lumière s'étend depuis l'extrémité proximale du cathéter de dilatation 120 jusqu'à un orifice 126 situé au niveau du coude 123. L'orifice 126 est disposé dans la partie convexe de manière à être dans le prolongement de la lumière pratiquée dans la portion amont 121. Cette première lumière 141 est ainsi sensiblement rectiligne. Le cathéter de dilatation 120 peut coulisser le long du premier guide 5. Ce dernier assure par conséquent une fonction de guidage pour le cathéter de dilatation 120. L'extrémité proximale du premier guide 5 est destinée à dépasser de l'extrémité proximale du cathéter de dilatation 120. L'extrémité distale du premier guide 5 est destinée à dépasser de l'orifice 126. Ainsi lorsque la partie distale du cathéter de dilatation 120 est introduite dans le vaisseau 1 et que la partie convexe du coude 123 est orientée vers l'amont du vaisseau 1, le guide s'échappe lui aussi du cathéter de dilatation 120 en direction de l'amont du vaisseau 1.

Quand le premier guide 5 est maintenu au niveau de son extrémité proximale, et que l'opérateur tire ou pousse l'extrémité proximale du cathéter de dilatation 120 en direction du passage transpariétal, la partie distale du cathéter de dilatation 120 peut coulisser le long de ce premier guide vers son extrémité proximale ou distale dans le vaisseau 1. Le cathéter de dilatation 120 selon l'invention permet d'être positionné de manière particulièrement précise et simple le long du vaisseau 1. Ainsi l'invention permet d'améliorer significativement la précision du contrôle de la position de l'ensemble du dispositif d'implantation. Le stent 130 et la prothèse 110 sont par conséquent mieux positionnés par rapport au vaisseau 1. La stabilité et l'étanchéité entre la prothèse 110 et le vaisseau 1 sont ainsi renforcées. L'invention permet également de réduire le temps nécessaire aux opérations d'implantation de la prothèse.

De manière préférée le cathéter de dilatation 120 est suffisamment souple de sorte qu'il puisse être déformé de façon à ce que la partie aval puisse sous l'effet d'une contrainte être disposée sensiblement dans le prolongement de la partie amont. Avantageusement, cette flexibilité au niveau du coude 123 facilite l'alignement approximatif des portions amont et aval 121, 122.

Le cathéter de dilatation 120 comporte une deuxième lumière 142 apte à accueillir un second guide 6. Cette deuxième lumière s'étend depuis l'extrémité proximale jusqu'à l'extrémité distale 124 du cathéter de dilatation 120. Le cathéter de dilatation 120 est agencé de manière à coulisser le long du second guide 6. Ce dernier assure par conséquent une fonction de guidage au cathéter de dilatation 120. Le second guide 6 sera par exemple de type guide souple.

Le second guide 6 présente une extrémité proximale destinée à dépasser de l'extrémité proximale du cathéter de dilatation 120. L'extrémité distale du second guide 6 s'échappe quand à elle par l'extrémité distale 124 du cathéter de dilatation 120. Ainsi lorsque la partie distale du cathéter de dilatation 120 est introduite dans le vaisseau 1 et que la partie convexe du coude 123 est orientée vers l'amont du vaisseau 1, le second guide 6 s'échappe du cathéter de dilatation 120 en direction de l'aval du vaisseau 1. Quand le second guide 6 est maintenu au niveau de son extrémité proximale et que l'opérateur tire ou pousse l'extrémité proximale du cathéter de dilatation 120 en direction du passage transpariétal, la partie distale de ce dernier peut alors coulisser le long du guide 6 dans le vaisseau 1 et respectivement vers l'aval ou l'amont de ce dernier.

Par conséquent, une poussée exercée par i'opérateur sur le cathéter de dilatation 120 permet de déplacer la portion aval 122 du cathéter de dilatation 120 le long du vaisseau 1 et respectivement vers l'amont de ce dernier, en glissant le long du premier guide 5 et du second guide 6.

De même, une traction exercée par l'opérateur sur le cathéter de dilatation 120 permet de déplacer la portion aval 122 du cathéter de dilatation 120 le long du vaisseau 1 et respectivement vers l'aval de ce dernier, en glissant le long du premier guide 5 et du second guide 6.

L'utilisation de deux guides 5,6 distincts, disposés respectivement en amont et en aval de la ponction, permet d'ajuster la position de l'implant de manière particulièrement précise tant en amont qu'en aval de !a ponction. Elle améliore également la sécurité du guidage.

En outre la présence des deux guides permet de mieux contrôler les positions au sein du vaisseau du coude et de l'extrémité distale du cathéter de dilatation. Ainsi, on réduit significativement le risque que le coude ou que l'extrémité distale du cathéter de dilatation ne s'intercale entre la paroi interne du vaisseau et une plaque d'athérome qui se serait fixée sur cette dernière.

La flexibilité du coude 123 permet de pouvoir retirer le cathéter de dilatation 120 hors du vaisseau 1 en le faisant coulisser dans le cathéter d'introduction 10 lorsque l'opérateur tire sur l'extrémité proximale du cathéter de dilatation 120. Plus précisément lorsque le cathéter de dilatation 120 est tiré vers son extrémité proximale il coulisse le long du premier 5 et/ou second 6 guide, entraînant un retour de la portion amont 121 dans le cathéter d'introduction 10 jusqu'au coude 123. Le coude 123 se déforme en s'ouvrant jusqu'à ce que la portion aval 122 s'aligne approximativement avec le cathéter d'introduction 10 pour pouvoir coulisser dans ce dernier. L'invention permet ainsi un retrait particulièrement simple, efficace et sûr du cathéter de dilatation 120 après implantation de la prothèse 110.

Par ailleurs, la flexibilité du cathéter de dilatation 120 permet de ramener la portion aval 122 suffisamment proche de la portion amont 121 de telle sorte que le ballon 128 en position non déployé puisse être simultanément en contact de la portion amont 121 et de la portion aval 122. Ainsi la flexibilité du coude 123 permet de replier le cathéter de dilatation 120 de manière à limiter considérablement son encombrement. Le cathéter de dilatation 120 peut ainsi être introduit dans des cathéters de largage conventionnels et de section limitée.

Avantageusement, en plus d'être aisément flexible le cathéter de dilatation 120 présente une très bonne élasticité. Ainsi, dès lors qu'il est évacué du cathéter de largage 30, il ne se trouve plus en position comprimée et retrouve automatiquement sa configuration de repos.

L'invention ne nécessite par conséquent aucun mécanisme spécifique pour ouvrir angulairement le cathéter de dilatation 120 lorsque son extrémité distale est extraite du cathéter de largage 30.

De même cette élasticité permet à la portion amont 121 de conserver une forme sensiblement rectiligne après avoir été déformée au niveau de l'extrémité distale 12 du cathéter d'introduction 10.

En outre, cette élasticité permet à la partie distale du cathéter de dilatation 120 de s'orienter approximativement selon la direction d'écoulement du sang.

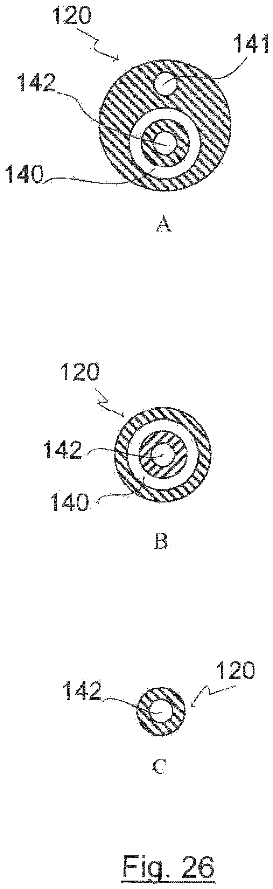

La figure 26A représente une vue en coupe transversale du cathéter 120 prise en amont du coude 123. Le cathéter 120 présente une structure sensiblement circulaire. Il comprend une lumière 140 de passage du fluide de dilatation, une lumière 141 de passage du premier guide 5 et une lumière 142 de passage du second guide 6. La lumière 142 de passage du second guide 6 est disposée au centre du cathéter 120. La lumière 140 de passage du fluide de dilatation est disposée de manière concentrique à la lumière 142 de passage du second guide 6. La lumière 141 de passage du premier guide 5 est disposée entre la lumière 140 de passage du fluide de dilatation et la périphérie du cathéter 120.

La figure 26B représente une vue en coupe transversale du cathéter 120 prise selon une section située en aval du coude 123 et à l'intérieur du ballon 128. Le diamètre du cathéter est moindre puisqu'il n'y a plus besoin de loger la lumière 141 de passage du premier guide 5. Seules subsistent les lumières 140 et 142.

La figure 26C représente une vue en coupe transversale du cathéter 120 prise selon une section située en aval de l'orifice de sortie par lequel le fluide de dilatation pénètre à l'intérieur du ballon pour le déployer. Ainsi, au niveau de cette section, le cathéter de dilatation 120 ne fait plus apparaître que la lumière de passage 142 du second guide 6.

A titre d'exemple non limitatif, un système d'implantation selon un mode de réalisation de l'invention va maintenant être détaillé.

Le cathéter d'introduction 10 présente un diamètre externe de 12 millimètres et un diamètre interne de 10,6 millimètres. L'extrémité distale 12 du cathéter d'introduction 10 présente un biseau taillé à 30°. La gorge 14 est située à 3 millimètres de cette même extrémité distale. La gorge 14 présente une longueur de 3 millimètres selon la direction longitudinale du cathéter d'introduction 10 et une profondeur de 0,3 millimètres. Le bossage 13 présente une hauteur de 3 millimètres et est disposé à 6 millimètres de l'extrémité distale 12 du cathéter d'introduction 10.

Le cathéter de largage 30 présente un diamètre externe d'environ 10,5 millimètres.

Le stent 130 est de type auto extensible en alliage à mémoire de forme ou expansible par ballonnet et sa longueur est d'environ 30 millimètres. Le stent 130 peut être comprimé et maintenu à un diamètre inférieur à 6 millimètres par des liens. Sa longueur est comprise entre 32 à 34 mm.

La prothèse 110 présente typiquement un corps 111 de 14 à 24 millimètres de diamètre suivant le diamètre du vaisseau 1, un départ de pontage de 10 à 20 millimètres de diamètre et des jambes 112 de 5 à 10 millimètres de diamètre chacune.

La longueur utile du ballon, c'est-à-dire la longueur de la portion permettant d'impacter l'implant dans le vaisseau est supérieure à celle du stent. Le diamètre du ballon déployé est sensiblement identique à celui du stent. A titre d'exemple non limitatif, le ballon est réalisé en silicone, en polyéthylène, en polyuréthane, en latex ou avec des dérivés de ces matériaux.

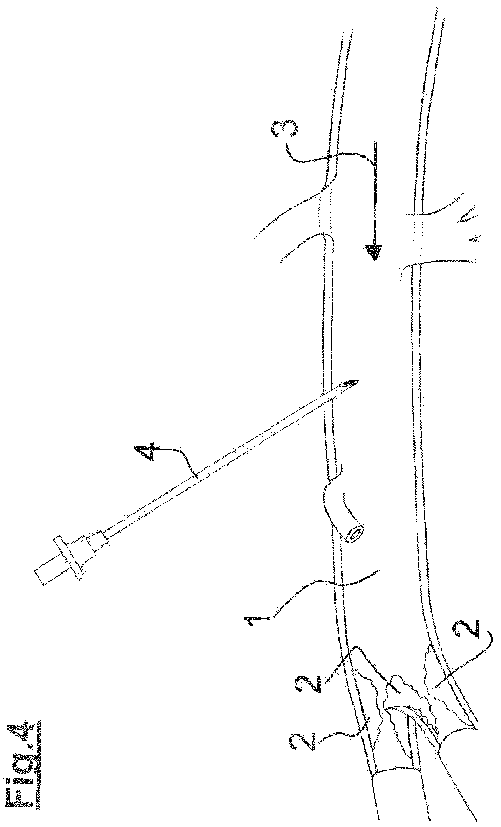

Les différentes étapes d'un procédé d'implantation d'une prothèse 110 selon l'invention vont maintenant être détaillées en références aux figures 4 à 20. Du sérum hépariné est injecté dans le cathéter d'introduction 10 avant usage et à différentes étapes du protocole d'implantation selon le souhait du clinicien.

Dans cet exemple, la prothèse 110 est destinée à assurer un pontage au niveau d'une sténose 2 présente dans une artère 1.

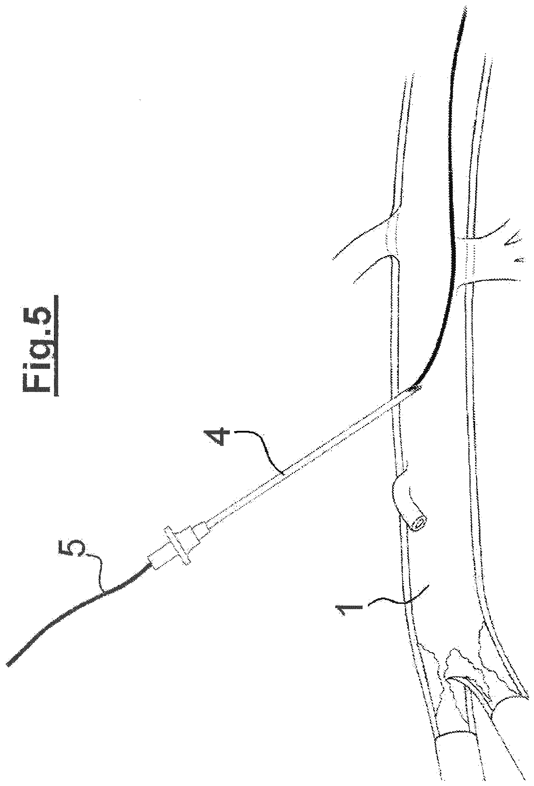

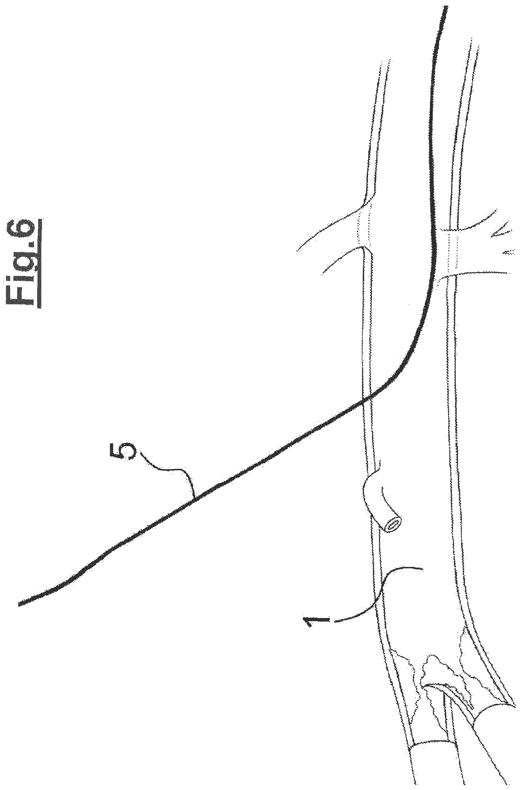

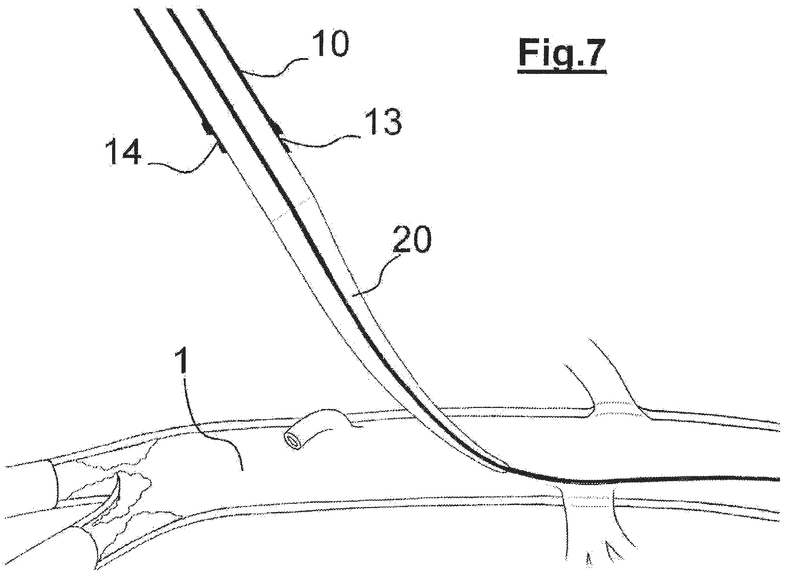

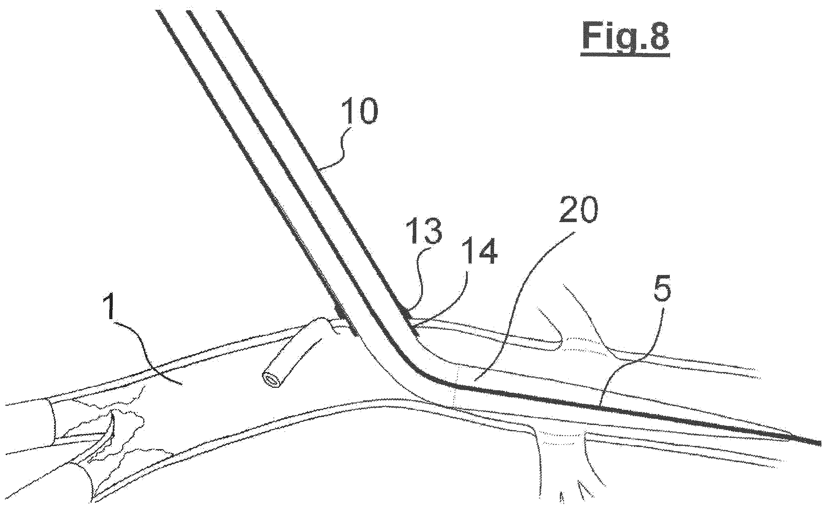

Comme représenté sur la figure 4, une ponction est réalisée dans l'artère grâce à une aiguille 4. Cette ponction définit alors au niveau de l'artère un passage transpariétal, l'aiguille 4 comprend une lumière et un premier guide 5 de type « guide stiff » est introduit dans l'artère par la lumière de l'aiguille 4 (figure 5). L'aiguille 4 est ensuite retirée et le premier guide 5 reste en place (figure 6). L'extrémité proximale du premier guide 5 est alors introduite dans l'extrémité distale du nez conique 22 du cathéter d'introduction 10. Le nez conique 20 du cathéter d'introduction 10 est ensuite amené au contact du vaisseau en glissant le long du premier guide 5. Le nez conique 20 du cathéter d'introduction 10 pénètre à travers la paroi du vaisseau 1 en glissant le long du premier guide 5 et élargit la section du passage transpariétal (figure 7). L'extrémité distale 12 du cathéter d'introduction 10 est également introduite dans le vaisseau 1 jusqu'à ce que son bossage 13 entre en butée avec la paroi externe du vaisseau. Le pourtour du passage transpariétal se loge alors dans la gorge 14 du cathéter d'introduction 10 (figure 8). La mise en position, le maintien en position et l'étanchéité entre le cathéter d'introduction 10 et le vaisseau sont ainsi assurés.

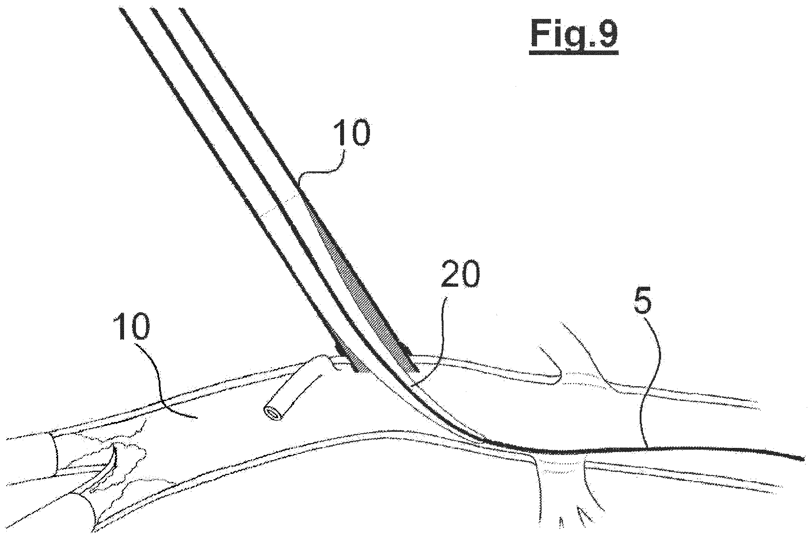

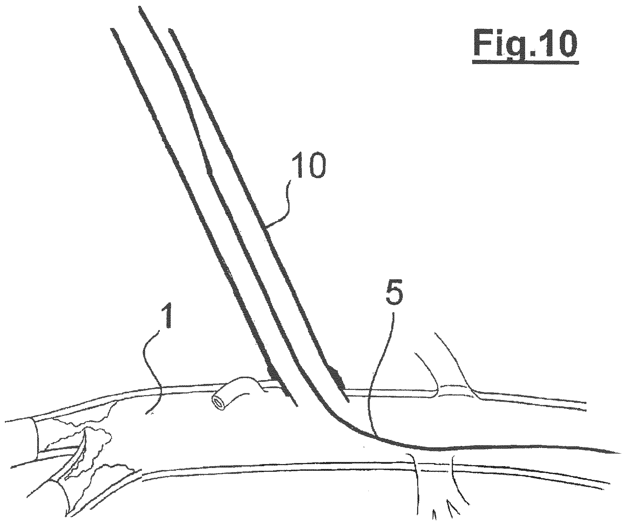

Le nez conique 20 est ensuite retiré du cathéter d'introduction 10 à travers la valve hémostatique et le long du guide qui reste quant à lui en position (figure 9). Du sérum hépariné est injecté dans le cathéter d'introduction 10 (figure 10).

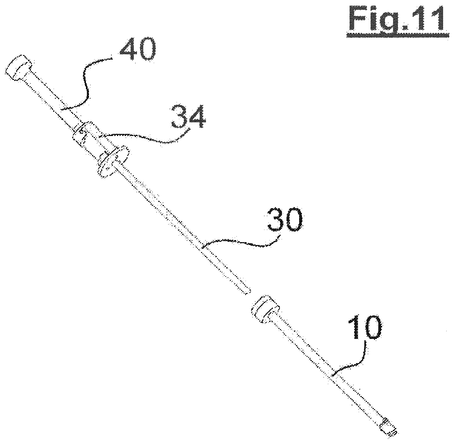

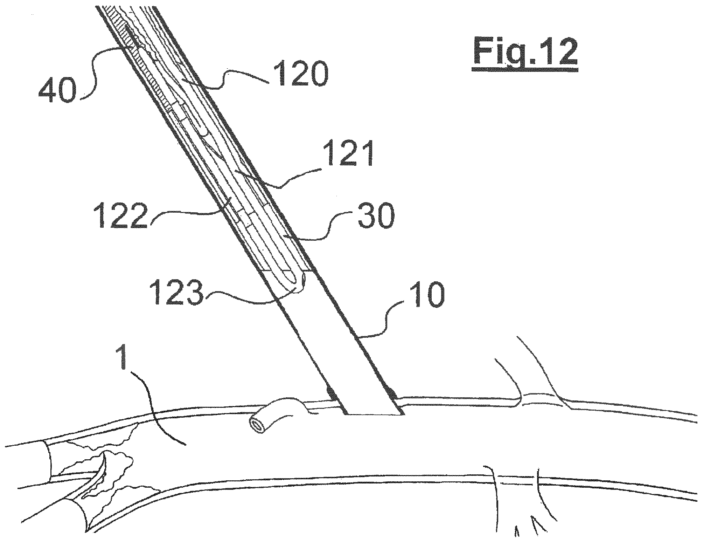

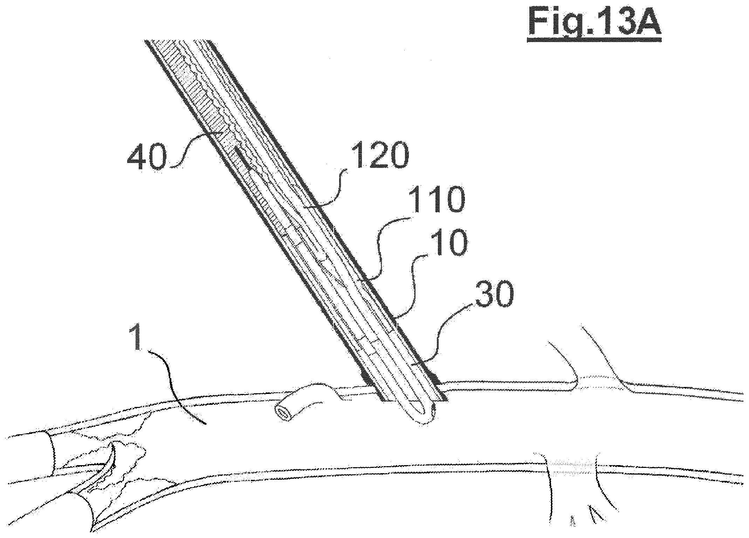

Le cathéter de largage 30, le dispositif de pousse 40 et l'implant sont préalablement mouillés de sérum hépariné. L'extrémité proximale du premier guide 5, qui dépasse de l'extrémité proximale 11 du cathéter d'introduction 10 est introduite dans l'orifice 126 du cathéter de dilatation 120 situé au niveau de son coude 123. Cet orifice 126 est accessible depuis l'extrémité distale du cathéter de largage 30. Le cathéter de largage 30 est introduit dans le cathéter d'introduction 10 (figures 11 et 12) et est avancé jusqu'à entrer en butée 33 avec l'extrémité proximale 11 du cathéter d'introduction 10. L'extrémité coudée du cathéter de dilatation 120 est alors légèrement protubérante dans l'artère (figure 13 A).

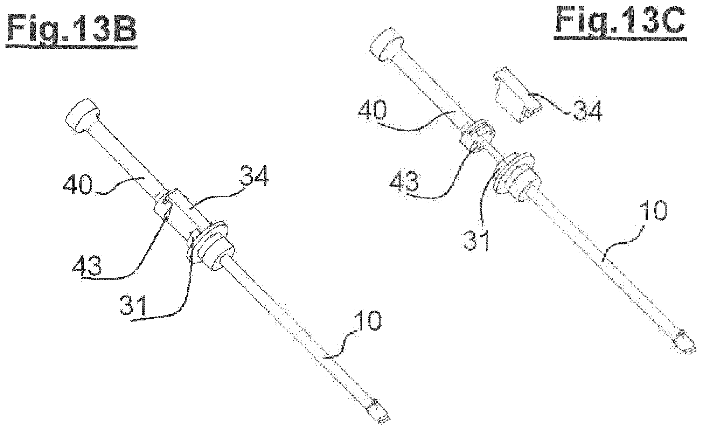

La cale 34 bloquant le dispositif de pousse 40 dans le cathéter de largage 30 est retirée (figures 13 B et 13 C). Le dispositif de pousse 40 est avancé dans le cathéter de largage 30 jusqu'à ce que la butée 43 du système de pousse entre au contact de l'extrémité proximale 31 du cathéter de largage 30. Le rôle de la cale 34 est clairement illustré sur les figures 13A à 14.

Le dispositif d'implantation est ainsi avancé le long du premier guide 5 par le dispositif de pousse 40. Ceci a pour effet d'avancer le corps 111 de la prothèse 110 dans le vaisseau 1 de manière à ce que le corps 111 de la prothèse 110 et le ballon 128 pénètrent dans l'artère.

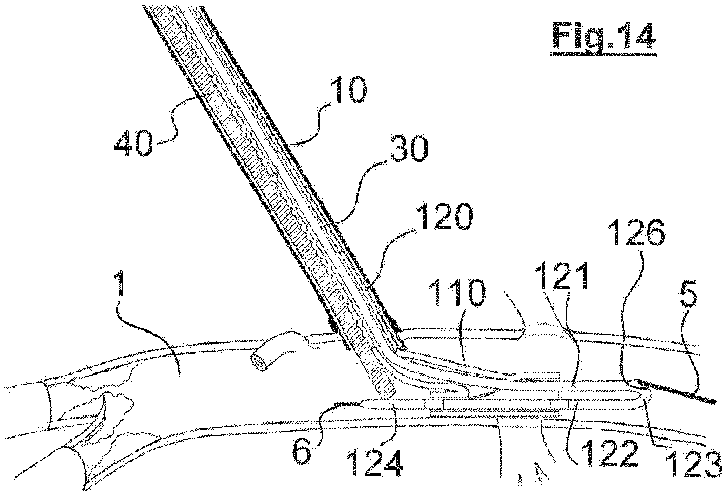

La portion aval 122 est alors libérée en rotation. Cette ouverture peut être subordonnée à l'ouverture d'un lien. Le stent 130 se positionne alors approximativement selon l'orientation du flux sanguin donc de l'axe principal du vaisseau (figure 14).

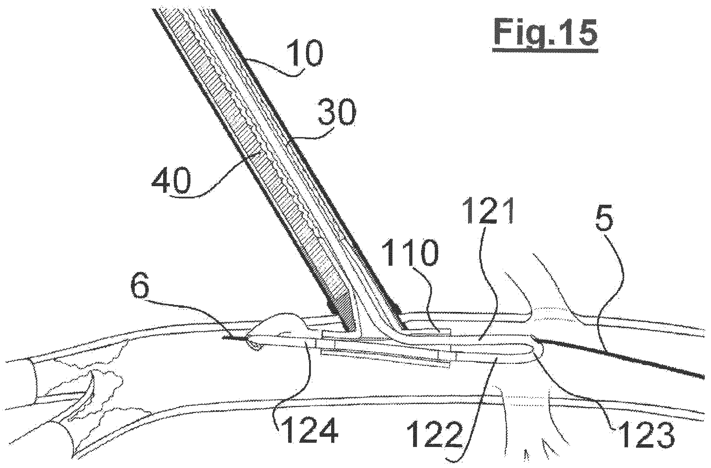

Sous contrôle radio, le cathéter de largage 30 est retiré vers l'arrière du cathéter d'introduction 10. Ce retrait peut par exemple être effectué sur une longueur de 12 millimètres et être contrôlé par un repère visuel disposé sur la partie tubulaire proximale du cathéter de largage 30. Ceci a pour effet d'amener la partie de l'implant contenant le stent 130 en butée contre l'extrémité distale 12 du cathéter d'introduction 10 (figure 15). Le stent 130 se positionne alors sous et en travers de l'extrémité distale 12 du cathéter d'introduction 10. Le bon positionnement du stent 130 est contrôlé par radio.

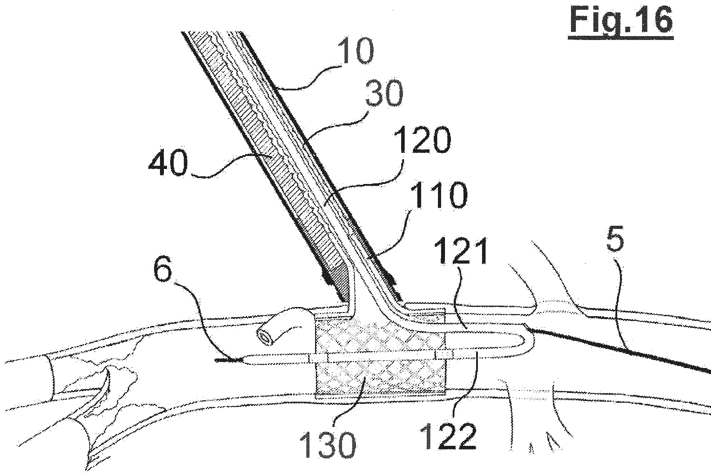

Sous contrôle radio également, le stent 130 est déployé par libération de ces liens et / ou par inflation du ballon. Le dispositif d'implantation n'est alors plus lié mécaniquement au dispositif de pousse. En poussant ou en tirant l'extrémité proximale du cathéter de dilatation 120 selon la direction du passage transpariétal, le ballon 128 peut être déplacé selon la direction principale 3 de l'artère et vers respectivement l'amont et l'aval de cette dernière en coulissant le long du premier guide 5 ou du second guide 6 (figure 16).

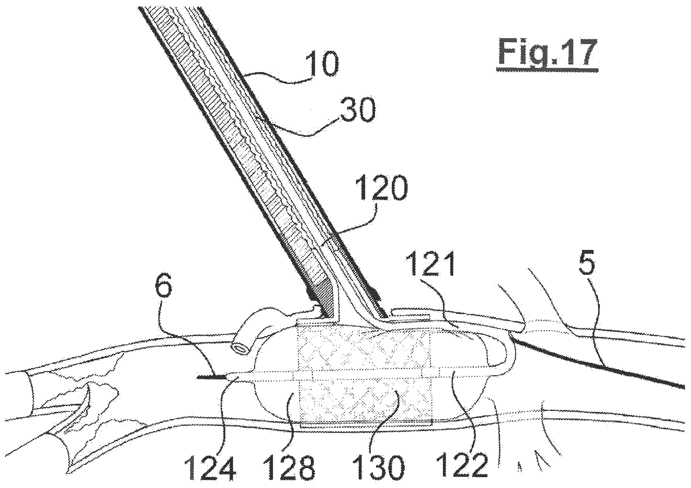

Sous contrôle radio, le ballon 128 est gonflé. Ceci a pour effet d'impacter l'implant dans le vaisseau et de pousser l'extrémité distale 12 du cathéter d'introduction 10 hors de l'artère (figure 17). A partir de cet instant l'implant assure l'étanchéité entre la prothèse vasculaire 110 et l'artère. Le positionnement du ballon selon la direction principale 3 peut à nouveau être ajusté en poussant ou en tirant l'extrémité proximale du cathéter de dilatation 120 de manière à faire coulisser le ballon 128 le long du premier guide 5 ou du second guide 6 et vers l'amont ou vers l'aval.

Ce nouveau positionnement du ballon suivi d'un nouveau gonflage de celui-ci peut optimiser l'impactage de l'implant dans le vaisseau et ainsi améliorer l'étanchéité.

Le cathéter d'introduction 10, le cathéter de largage 30 et le système de pousse sont ensuite retirés du patient.

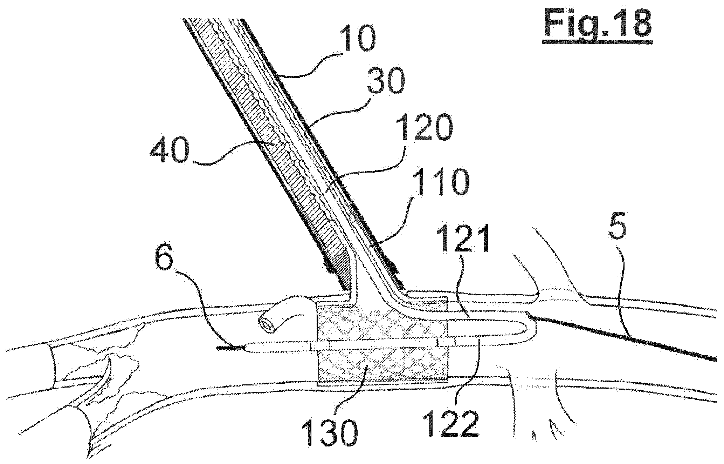

Le ballon 128 est ensuite dégonflé (figure 18). L'étanchéité de la jonction entre le dispositif d'implantation et le vaisseau est contrôlée. Si cette dernière n'est pas satisfaisante, le ballon 128 peut être regonflé pour une nouvelle tentative. En cas de problème persistant un clamp prépositionné en amont permet d'arrêter la circulation sanguine en amont de la ponction du vaisseau et un pontage classique peut être réalisé.

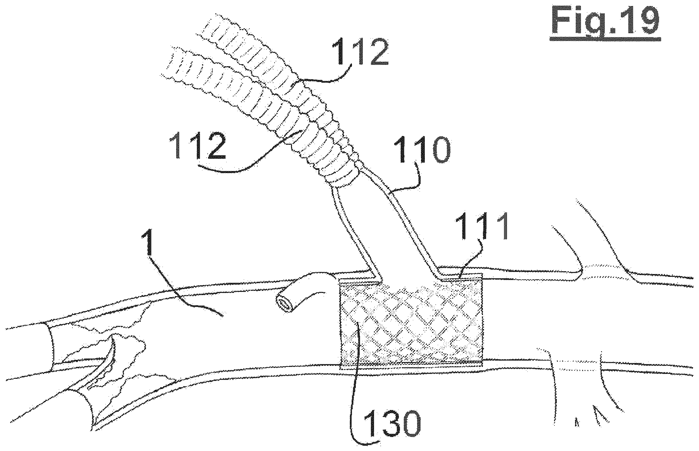

Une fois l'étanchéité de la jonction entre le dispositif d'implantation et le vaisseau satisfaisante, le cathéter de dilatation 120 est retiré en tirant sur son extrémité proximale. Le cathéter de dilatation 120 coulisse le long du premier guide 5 lors de ce retrait. Le premier guide 5 lui-même est ensuite retiré par la lumière de la prothèse 110 (figure 19).

La pose de l'implant est achevée et le pontage peut être terminé de manière conventionnelle.

L'exemple de réalisation précédent décrit un système d'implantation comprenant un premier guide 5 parcourant le vaisseau en amont de la ponction et un deuxième guide 6 parcourant le vaisseau en aval de la ponction. On pourra bien entendu prévoir que le premier guide 5 soit disposé en aval de la ponction et que le second guide 6 soit disposé en amont de la ponction.

Par ailleurs, dans un autre mode de réalisation, on prévoit un système d'implantation ne comportant qu'une seule lumière apte à accueillir un seul guide. Ce guide parcourt le vaisseau en amont ou en aval de la ponction et assure en collaboration avec l'unique lumière la même fonction que les lumières et les guides présentés précédemment : il permet de guider en coulissement la partie distale du cathéter de dilatation vers l'aval ou l'amont du vaisseau afin d'ajuster la position de l'implant.

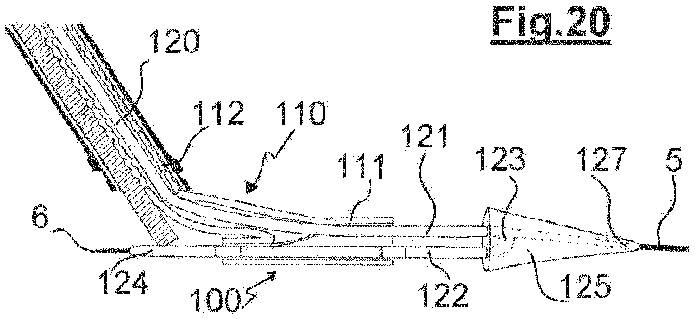

Une variante de réalisation du cathéter de dilatation 120 selon l'invention va maintenant être présentée en référence à la figure 20.

Le dispositif d'implantation présente au niveau du coude 123 un embout 125 de forme profilée prolongeant le cathéter de dilatation 120. Cet embout 125 profilé présente une configuration sensiblement tronconique. Il comporte une extrémité obtuse 127 et une extrémité évasée. Le diamètre de l'embout 125 se rétrécit dans le prolongement du coude 123 et s'évase depuis le coude 123 vers l'extrémité distale 124 du cathéter de dilatation 120. L'embout 125 enveloppe le coude 123. Il comporte une lumière coïncidant avec l'orifice 126 de manière à ce que la première lumière du cathéter de dilatation 120 coïncide avec la lumière de l'embout 125. Ainsi le premier guide 5 assure un guidage par coulissement du cathéter de dilatation 120 et de l'embout 125.

A titre d'exemple non limitatif, la longueur de l'extrémité obtuse est comprise entre 3 et 12cm. L'extrémité obtuse présente une pente comprise entre 5° et 25°. Le matériau de l'embout est en plastique. Avantageusement, l'angle au sommet du cône est compris entre 10° et 50°.

Cet exemple de réalisation permet de se passer du nez conique 20 et du cathéter de largage 30 de l'exemple de réalisation précédemment décrit.

L'embout 125 fait office de moyens de pénétration. Il fait saillie de l'extrémité distale 12 du cathéter d'introduction 10. Il est guidé par le premier guide 5 jusqu'à la ponction pratiquée dans la paroi du vaisseau 1. Il pénètre à travers cette ponction et élargit la section de cette dernière. Il permet au pourtour du passage transpariétal de se loger dans les moyens d'étanchéité et de stabilisation 16 du cathéter d'introduction 10. Puisque l'embout 125 fait office de moyens de pénétration, un tel cathéter d'introduction 10 permet de supprimer l'étape consistant à introduire un nez conique 20 dans le cathéter d'introduction 10 et à l'en extraire ultérieurement. Un tel cathéter de dilatation 120 permet ainsi de simplifier les procédés conventionnels d'implantation de prothèse 110.

En outre un tel cathéter de dilatation 120 est logé dans un cathéter de largage 30 qui peut être inséré dans le cathéter d'introduction 10 dès le début de l'opération. Il réduit par conséquent significativement le nombre d'étapes et la durée des procédés habituels d'implantation.

De plus l'embout 125 demeure constamment au devant de la prothèse 110 et du stent 130 lors de l'introduction de ces derniers dans le vaisseau 1. Il contribue par conséquent à protéger efficacement l'intégrité de l'implant et du vaisseau 1.

De manière préférée on prévoit que l'extrémité distale 124 du cathéter de dilatation 120 comporte un second embout 125. Ce second embout 125 présente également une forme sensiblement tronconique. Le second embout 125 comporte une extrémité obtuse 127 prolongeant l'extrémité distale 124 du cathéter de dilatation 120 et une extrémité évasée s'évasant depuis l'extrémité distale 124 du cathéter de dilatation 120 en direction du coude 123. Les extrémités évasées des deux embouts 125 sont disposées en regard.

De manière alternative, l'extrémité obtuse 127 de chaque embout 125 peut être sphérique et non conique. Cette forme sphérique procure sensiblement les mêmes avantages que les formes coniques précédemment décrites.

Notamment, le second embout 125 protège le stent 130, la prothèse 110 et le vaisseau 1 lors du recul du dispositif d'implantation.

Les embouts 125 peuvent être sensiblement rigides. A cet effet, la prothèse 110 présente une ouverture dans la partie du pontage avant la bifurcation pour pouvoir retirer le cathéter de dilatation 120. Une fois la prothèse 110 mise en place, le pontage peut être clampé juste au dessus de l'artère, la circulation sanguine n'est alors pas arrêtée au niveau du circuit vasculaire du patient et le chirurgien peut suturer cette ouverture. De manière préférée, cette ouverture peut comporter une suture détendue, un lac ou un autre système à activer pour refermer instantanément l'ouverture après le retrait du ballon 128.

Dans un autre mode de réalisation, les embouts 125 sont agencés de manière à ce que leur section selon un plan sensiblement perpendiculaire à la direction longitudinale de la portion aval 122 puisse être réduite de manière commandée. Cette réduction de section permet de retirer le cathéter de dilatation 120 à travers les conduits formés par les jambes 112 de la prothèse 110 sans avoir à pratiquer d'ouverture dans cette dernière.

Cette réduction de section peut être obtenue par traction mécanique. Elle peut également être obtenue en retirant le fluide alimentant les embouts 125. A cet effet on prévoit que le cathéter de dilatation 120 comporte un conduit agencé de manière à alimenter en fluide chacun des embouts 125 profilés pour obtenir des sections d'embouts variables à volonté selon l'alimentation ou le retrait de fluide.

A titre d'exemple non limitatif, un procédé d'implantation d'une prothèse 110 vasculaire effectué au moyen d'un dispositif d'implantation représenté en figure 20 peut comprendre les étapes suivantes :
- on réalise une ponction dans le vaisseau 1 à implanter à l'aide d'une aiguille 4.
- on introduit un premier guide 5 à travers l'aiguille 4 que l'on retire.
- on fait coulisser le long du premier guide 5 un cathéter d'introduction 10 duquel fait saillie au niveau de son extrémité distale l'embout 125 enveloppant le coude 123 du cathéter de dilatation 120 logé dans le cathéter d'introduction 10.
- l'embout 125 pénètre dans la ponction depuis son extrémité obtuse 127 et élargit la section de cette dernière au fur et à mesure de son insertion.
- on introduit l'embout 125 dans le vaisseau 1 jusqu'à ce que l'extrémité distale 12 du cathéter d'introduction 10 pénètre à son tour dans ce dernier et que les moyens d'étanchéité et de stabilisation 16 coopèrent avec le pourtour du passage transpariétal.
- on introduit l'embout 125 et la partie distale du cathéter de dilatation 120 dans le vaisseau 1 en poussant le dispositif d'implantation en direction de l'extrémité distale 32 du cathéter d'introduction 10.
- les étapes suivantes sont similaires à celles prévues dans un procédé utilisant le dispositif d'implantation précédemment décrit et illustré sur les figures 4 à 19. Les portions amont 11 et aval 12 du cathéter d'introduction 10 sont libérées en rotation.
- la portion aval 122 s'oriente de manière naturelle selon la direction d'écoulement du flux sanguin.
- on ajuste la position du cathéter de dilatation 120 vers l'amont ou vers l'aval du vaisseau 1 en le faisant coulisser le long respectivement du premier guide 5 ou du second guide 6.
- on gonfle le ballon 128 de manière à impacter l'implant dans le vaisseau.
- on dégonfle le ballon 128 et on retire ce dernier ainsi que le cathéter de dilatation 120 hors du cathéter d'introduction 10.
- on retire le cathéter d'introduction 10.

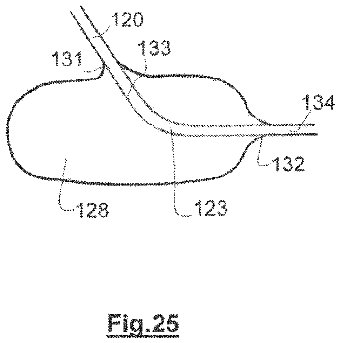

Selon une variante de réalisation illustrée en Figure 25 et ne comprenant pas l'invention, le cathéter de dilatation présente un coude 123 logé à l'intérieur d'un ballon 128 présentant une portion centrale sensiblement cylindrique délimitée par deux extrémités. Le dispositif d'implantation est agencé de manière à ce qu'un point de liaison 131 entre le ballon 128 et le cathéter de dilatation 120 soit situé sensiblement au milieu du cylindre et de manière à ce qu'un autre point 132 de liaison entre le ballon 128 et e cathéter de dilatation 120 soit situé sensiblement à l'une des extrémités du ballon 128.

Ainsi, le coude 123 présente sensiblement une forme de « L ». De manière plus générale les portions adjacentes 133, 134 au coude 123 forment un angle compris entre 130° et 60° et plus particulièrement un angle compris entre 120° et 90°.

La portion de cathéter de dilatation située entre le coude et l'extrémité distale du cathéter de dilatation est désignée portion aval 134. La portion de cathéter de dilatation adjacente au coude 123 et située en amont du coude 123 est désignée portion amont 133. Ainsi, le ballon 128 est porté d'une part par la portion aval 134 et d'autre part par la portion amont 133. Il est donc partiellement porté par la portion aval 134. Le dispositif d'implantation est agencé de manière à ce que la portion aval 134 s'étende sensiblement parallèlement au vaisseau lors de l'implantation.

Avantageusement, le ballon 128 s'étend sensiblement sur la totalité de la longueur de la portion aval 134.

Le cathéter de dilatation comporte une lumière permettant le passage d'un guide depuis l'extrémité proximale jusqu'à l'extrémité distale du cathéter de dilatation. Ce guide, semblablement aux premier et second guides décrits dans les exemples précédents a pour fonction d'assurer le guidage en coulissement du cathéter de dilatation. Pour des raisons de clarté, ce guide n'est pas représenté sur la figure 25.

Cette variante de réalisation permet de réduire l'encombrement du dispositif d'implantation et de simplifier et sécuriser le procédé d'implantation de la prothèse. En effet, la portion du cathéter de dilatation dans laquelle circule le guide 2 n'a plus raison d'être ; sa suppression diminue l'encombrement du dispositif. Pour rappel, dans les modes de réalisation précédents, une étape consiste à pousser suffisamment l'implant hors du cathéter de langage pour introduire dans le vaisseau l'ensemble des portions amont et aval du cathéter de dilatation. Une étape ultérieure de recul est ensuite nécessaire pour ramener le milieu de l'implant sensiblement au droit de la ponction. Du fait de l'absence de la portion du cathéter de dilatation contenant la lumière dédiée au second guide, le dépassement du cathéter de dilatation de l'extrémité aval de l'implant est moindre. Ceci réduit l'amplitude des mouvements nécessaires à l'introduction de l'implant dans le vaisseau et à son recul pour le positionner sensiblement au droit de la ponction. Cette amplitude réduite de mouvements limite les risques liés au positionnement de l'implant dans le vaisseau.

Un tel dispositif d'implantation pourra bien entendu être combiné avec les variantes de réalisations décrites ci-dessus concernant les embouts profilés.

Les dimensions du dispositif d'implantation seront aisément adaptées pour convenir aux différents diamètres de vaisseaux à implanter. On notera que l'invention, en améliorant la simplicité et la sécurité de l'implantation d'une prothèse au droit d'une ponction s'avère particulièrement avantageuse pour les vaisseaux dont le diamètre est petit ou dont l'accès par voie intraluminale est difficile.

La présente invention offre ainsi plusieurs solutions concernant respectivement un cathéter de dilatation, des moyens d'étanchéité et de stabilisation du cathéter d'introduction et l'extrémité distale du cathéter d'introduction. Ces solutions, bien que techniquement indépendantes contribuent chacune à améliorer la précision et à diminuer la durée et la difficulté des procédés conventionnels d'implantation de prothèse vasculaire.

### REFERENCES

- 1.: Vaisseau
- 2.: Sténose
- 3.: Direction principale
- 4.: Aiguille
- 5.: Premier guide
- 6.: Second guide
- 10.: Cathéter d'introduction
- 11.: Extrémité proximale du cathéter d'introduction
- 12.: Extrémité distale du cathéter d'introduction
- 13.: Bossage
- 14.: Gorge
- 15.: Profilé
- 16.: Moyens d'étanchéité et de stabilisation
- 20.: Nez conique
- 21.: Extrémité proximale du nez conique
- 22.: Extrémité distale du nez conique
- 30.: Cathéter de largage
- 31.: Extrémité proximale du cathéter de largage
- 32.: Extrémité distale du cathéter de largage
- 33.: Butée du cathéter de largage
- 40.: Dispositif de pousse
- 41.: Extrémité proximale du dispositif de pousse
- 42.: Extrémité distale du dispositif de pousse
- 43.: Butée du système de pousse
- 34.: Entretoise
- 110.: Prothèse
- 111.: Corps
- 112.: Jambe
- 120.: Cathéter de dilatation
- 121.: Portion amont
- 122.: Portion aval
- 123.: Coude
- 124.: Extrémité distale du cathéter de dilatation
- 125.: Embout
- 126.: Orifice
- 127.: Extrémité obtuse
- 128.: Ballon
- 129.: Rainure
- 130.: Stent
- 131.: Premier point de liaison
- 132.: Deuxième point de liaison
- 133.: Portion amont du cathéter de dilatation
- 134.: Portion aval du cathéter de dilatation
- 140.: Lumière de passage du fluide de dilatation
- 141.: Lumière de passage du premier guide
- 142.: Lumière de passage du second guide

## Revendications

1. Système d'implantation d'une prothèse (110) dans un vaisseau (1) comportant :
- un cathéter d'introduction (10), comprenant une extrémité proximale (11) et une extrémité distale (12),
- un dispositif d'implantation pour implanter la prothèse (110) au sein d'un vaisseau (1), comportant un ballon (128) et un cathéter de dilatation (120) pour alimenter le ballon (128) en fluide, le cathéter de dilatation (120) comprenant une extrémité proximale, une extrémité distale (124) et une partie distale destinée à être introduite dans le vaisseau (1), **caractérisé en ce que** la partie distale comprend un coude (123) ainsi qu'une portion amont (121) et une portion aval (122) contiguës au coude (123) et disposées entre le coude (123) et respectivement l'extrémité proximale et l'extrémité distale (124), le ballon (128) étant entièrement porté par la portion aval (122),
le dispositif d'implantation étant destiné à coulisser depuis l'extrémité proximale (11) jusqu'à l'extrémité distale (12) du cathéter d'introduction (10),
le dispositif d'implantation comportant une prothèse (110) et/ou un stent (130) à l'intérieur duquel est logé le ballon (128).

2. Système d'implantation d'une prothèse (110) selon la revendication précédente, dans lequel le dispositif d'implantation est tel que les portions amont (121) et aval (122) forment un angle compris entre 0° et 30°.

3. Système d'implantation d'une prothèse (110) selon l'une quelconque des revendications précédentes, dans lequel le dispositif d'implantation est tel que le coude (123) présente sensiblement une forme de U.

4. Système d'implantation d'une prothèse (110) selon l'une quelconque des revendications précédentes, dans lequel le dispositif d'implantation est tel que le cathéter de dilatation (120) est élastique au niveau du coude (123) de manière à ce que les portions amont (121) et aval (122) puissent être mutuellement approchées ou écartées.

5. Système d'implantation d'une prothèse (110) selon l'une quelconque des revendications précédentes, dans lequel le dispositif d'implantation est tel que le cathéter de dilatation (120) comporte une première lumière s'étendant entre son extrémité proximale et le coude (123) et apte à recevoir un premier guide (5).

6. Système d'implantation d'une prothèse (110) selon l'une quelconque des revendications précédentes, dans lequel le dispositif d'implantation comporte une deuxième lumière s'étendant entre l'extrémité proximale et l'extrémité distale (124) du cathéter de dilatation (124), la deuxième lumière étant conformée pour recevoir un second guide (6).

7. Système d'implantation d'une prothèse (110) selon l'une quelconque des revendications précédentes, dans lequel le dispositif d'implantation est tel que le coude (123) et/ou l'extrémité distale (124) du cathéter de dilatation (120) présente un embout (125) profilé en forme de cône ou de sphère prolongeant la portion aval (122).

8. Système d'implantation d'une prothèse (110) selon l'une quelconque des revendications précédentes, **caractérisé en ce que** le cathéter d'introduction (10) comporte des moyens d'étanchéité et de stabilisation (16) agencés de manière à permettre une liaison stable et étanche entre le cathéter d'introduction (10) et le vaisseau (1).

9. Système d'implantation d'une prothèse (110) selon la revendication précédente, **caractérisé en ce que** les moyens d'étanchéité et de stabilisation comportent un bossage (13) et une gorge (14) disposée entre le bossage (13) et l'extrémité distale (12) du cathéter d'introduction (10).

10. Système d'implantation d'une prothèse (110) selon l'une quelconque des deux revendications précédentes, **caractérisé en ce que** les moyens d'étanchéité et de stabilisation (16) sont élastiques et agencés pour épouser les parois d'un orifice approximativement circulaire.

11. Système d'implantation d'une prothèse (110) selon l'une quelconque des revendications précédentes, dans lequel le dispositif d'implantation comporte une prothèse (110) et un stent (130) à l'intérieur duquel est logé le ballon (128) et dans lequel le stent est disposé entre la prothèse (110) et le ballon (128).

12. Système d'implantation d'une prothèse (110) selon l'une quelconque des revendications précédentes, comportant un cathéter de largage (30) apte à être inséré dans le cathéter d'introduction (10), le cathéter de largage (30) présentant une extrémité proximale (31), une extrémité distale (32) et un corps tubulaire s'étendant entre l'extrémité proximale (31) et l'extrémité distale (32), les sections extérieures de l'extrémité distale (32) et du corps tubulaire du cathéter de largage (30) étant agencées de manière à ce que ce dernier puisse coulisser à l'intérieur du corps du cathéter d'introduction (10) en étant introduit par une extrémité proximale (11) de ce dernier, dans lequel le cathéter de dilatation (120) et le ballon (128) sont positionnés dans le stent (130) et dans lequel le cathéter de dilatation (120), le ballon (128) et le stent (130) sont montés dans le cathéter de largage (30).

## Patentansprüche

1. System zur Implantation einer Prothese (110) in ein Gefäß (1), welches folgendes umfasst:
- einen Einführkatheter (10), welcher ein proximales Ende (11) und ein distales Ende (12) umfasst,
- eine Implantationsvorrichtung zum Implantieren der Prothese (110) in ein Gefäß (1), welche einen Ballon (128) und einen Dilatationskatheter (120) zur Versorgung des Ballons (128) mit einem Medium umfasst, wobei der Dilatationskatheter (120) ein proximales Ende, ein distales Ende (124) und einen distalen Teil umfasst, der dazu bestimmt ist, in das Gefäß (1) eingeführt zu werden, **dadurch gekennzeichnet, dass** der distale Teil ein Winkelstück (123) sowie einen vorderen Abschnitt (121) und einen hinteren Abschnitt (122) umfasst, die am Winkelstück (123) angrenzen und zwischen dem Winkelstück (123), und jeweils dem proximalen Ende und dem distalen Ende (124) angeordnet sind, wobei der Ballon (128) in vollem Umfang vom hinteren Abschnitt (122) getragen wird, wobei die Implantationsvorrichtung dazu bestimmt ist, vom proximalen Ende (11) bis zum distalen Ende (12) des Einführkatheters (10) zu gleiten, wobei die Implantationsvorrichtung eine Prothese (110) und/oder einen Stent (130) umfasst, in dem der Ballon (128) gelagert ist.

2. System zur Implantation einer Prothese (110) nach dem vorhergehenden Anspruch, in welchem die Implantationsvorrichtung so gestaltet ist, dass der vordere Abschnitt (121) und der hintere Abschnitt (122) einen Winkel von 0° bis 30° bilden.

3. System zur Implantation einer Prothese (110) nach einem der vorhergehenden Ansprüche, in welchem die Implantationsvorrichtung so gestaltet ist, dass das Winkelstück (123) im Wesentlichen eine U-Form hat.

4. System zur Implantation einer Prothese (110) nach einem der vorhergehenden Ansprüche, in welchem die Implantationsvorrichtung so gestaltet ist, dass der Dilatationskatheter (120) im Bereich des Winkelstücks (123) elastisch ist, so dass der vordere Abschnitt (121) und der hintere Abschnitt (122) einander angenähert oder voneinander entfernt werden können.

5. System zur Implantation einer Prothese (110) nach einem der vorhergehenden Ansprüche, in welchem die Implantationsvorrichtung so gestaltet ist, dass der Dilatationskatheter (120) ein erstes Schlitzloch umfasst, das sich zwischen seinem proximalen Ende und dem Winkelstück (123) erstreckt und in der Lage ist, eine erste Führung (5) aufzunehmen.

6. System zur Implantation einer Prothese (110) nach einem der vorhergehenden Ansprüche, in welchem die Implantationsvorrichtung, ein zweites Schlitzloch umfasst, das sich zwischen dem proximalen Ende und dem distalen Ende (124) des Dilatationskatheters (124) erstreckt, wobei das zweite Schlitzloch so angepasst ist, dass es eine zweite Führung (6) aufnehmen kann.

7. System zur Implantation einer Prothese (110) nach einem der vorhergehenden Ansprüche, in welchem die Implantationsvorrichtung so gestaltet ist, dass das Winkelstück (123) und/oder das distale Ende (124) des Dilatationskatheters (120) ein profiliertes konus- oder kugelförmiges Endstück (125) aufweist, das den hinteren Abschnitt (122) verlängert.

8. System zur Implantation einer Prothese (110) nach einem der vorhergehenden Ansprüche, **dadurch gekennzeichnet, dass** der Einführkatheter (10) Dichtungs- und Stabilisierungsmittel (16) umfasst, die so angeordnet sind, dass die Herstellung einer stabilen und dichten Verbindung zwischen dem Einführkatheter (10) und dem Gefäß (1) möglich ist.

9. System zur Implantation einer Prothese (110) nach dem vorhergehenden Ansprüche, **dadurch gekennzeichnet, dass** die Dichtungs- und Stabilisierungsmittel einen Ansatz (13) und eine Nut (14) zwischen dem Ansatz (13) und dem distalen Ende (12) des Einführkatheters (10) umfassen.

10. System zur Implantation einer Prothese (110) nach dem zwei vorhergehenden Ansprüche, **dadurch gekennzeichnet, dass** die Dichtungs- und Stabilisierungsmittel (16) elastisch sind und so angeordnet sind, dass sie sich an die Wände einer annähernd kreisförmigen Öffnung anlegen.

11. System zur Implantation einer Prothese (110) nach einem der vorhergehenden Ansprüche, in welchem die Implantationsvorrichtung eine Prothese (110) aufweist und einen Stent (130), in dem der Ballon (128) gelagert ist, und in dem der Stent zwischen Prothese (110) und Ballon (128) angeordnet ist.

12. System zur Implantation einer Prothese (110) nach einem der vorhergehenden Ansprüche, welches einen Zubehör-Einführkatheter (30) umfasst, der in den Einführkatheter (10) eingeführt werden kann, wobei der Zubehör-Einführkatheter (30) ein proximales Ende (31), ein distales Ende (32) und einen röhrenförmigen Körper aufweist, der sich zwischen dem proximalen Ende (31) und dem distalen Ende (32) erstreckt, wobei die äußeren Abschnitte des distalen Endes (32) und des röhrenförmigen Körpers des Zubehör-Einführkatheters (30) so angeordnet sind, dass letzterer in dem Körper des Einführkatheters (10) gleiten kann, indem er durch ein proximales Ende (11) des letzteren eingeführt wird, in welchem der Dilatationskatheter (120) und der Ballon (128) im Stent (130) positioniert sind und in welchem der Dilatationskatheter (120), der Ballon (128) und der Stent (130) in den Zubehör-Einführkatheter (30) montiert sind.

## Claims

1. A system for implanting a prosthesis (110) in a vessel (1) including :
- an insertion catheter (10) comprising a proximal end (11) and a distal end (12),
- an implantation device for implanting the prosthesis (110) in a vessel (1), including a balloon (128) and a dilatation catheter (120) for supplying the balloon (128) with fluid, with the dilatation catheter (120) comprising a proximal end, a distal end (124) and a distal portion intended to be introduced into the vessel (1), **characterized in that** the distal portion comprises a bend (123) as well as an upstream portion (121) and a downstream portion (122) adjacent to the bend (123) and positioned between the bend (123) and the proximal end and the distal end (124) respectively, with the balloon (128) being integrally carried by the downstream portion (122),
the implantation device being intended to slide from the proximal end (11) to the distal end (12) of the insertion catheter (10),
the implantation device including a prosthesis (110) and/or a stent (130) inside which the balloon (128) is accommodated.

2. A system for implanting a prosthesis (110) according to the preceding claim, wherein the implantation device is such that the upstream (121) and the downstream (122) portions form an angle between 0° and 30°.

3. A system for implanting a prosthesis (110) according to any one of the preceding claims, wherein the implantation device is such that the bend (123) has a substantially U-shape.

4. A system for implanting a prosthesis (110) according to any one of the preceding claims, wherein the implantation device is such that the dilatation catheter (120) is elastic at the bend (123) so that the upstream (121) and downstream (122) portions can be mutually approached or spaced apart.

5. A system for implanting a prosthesis (110) according to any one of the preceding claims, wherein the implantation device is such that the dilatation catheter (120) comprises a first aperture extending between the proximal end and the bend (123) thereof and being adapted to receive a first guide (5).

6. A system for implanting a prosthesis (110) according to any one of the preceding claims, wherein the implantation device includes a second aperture extending between the proximal end and the distal end (124) of the dilatation catheter (124), with the second aperture being so shaped as to receive a second guide (6).

7. A system for implanting a prosthesis (110) according to any one of the preceding claims, wherein the implantation device is such that the bend (123) and/or the distal end (124) of the dilatation catheter (120) has an end piece (125) having the shape of a cone or of a sphere expanding the downstream portion (122).

8. A system for implanting a prosthesis (110) according to any one of the preceding claims, **characterized in that** the insertion catheter (10) includes sealing and stabilizing means (16) so arranged as to enable a stable and sealed bond between the insertion catheter (10) and the vessel (1).

9. A system for implanting a prosthesis (110) according to the preceding claim, **characterized in that** the sealing and stabilizing means include a boss (13) and a groove (14) positioned between the boss (13) and the distal end (12) of the insertion catheter (10).

10. A system for implanting a prosthesis (110) according to any one of the preceding claims, **characterized in that** the sealing and stabilizing means (16) are elastic and so arranged as to conform to the walls of a substantially circular hole.

11. A system for implanting a prosthesis (110) according to any one of the preceding claims, wherein the implantation device includes a prosthesis (110) and a stent (130) inside which the balloon (128) is accommodated and wherein the stent is positioned between the prosthesis (110) and the balloon (128).

12. A system for implanting a prosthesis (110) according to any one of the preceding claims, including a delivery catheter (30) adapted to be inserted into the insertion catheter (10), with the delivery catheter (30) having a proximal end (31), a distal end (32) and a tubular body which extends between the proximal end (31) and the distal end (32), with the outer sections of the distal end (32) and of the tubular body of the delivery catheter (30) being so arranged that the latter can slide inside the body of the insertion catheter (10) by being introduced through a proximal end of the latter, wherein the dilatation catheter (120) and the balloon (128) are positioned in the stent (130) and wherein the dilatation catheter (120), the balloon (128) and the stent (130) are mounted in the delivery catheter (30).
